# EUROPEAN PATENT SPECIFICATION

(11) **EP 4 400 562 B1**
(45) Date of publication and mention of the grant of the patent: **05.11.2025**
(21) Application number: 22889661.9
(22) Date of filing: 13.09.2022
(51) Int. Cl.: C10B 57/00, C10B 57/04, G01N 33/22, G01N 5/04

(54) **METHOD FOR ESTIMATING COKE STRENGTH AFTER REACTION AND METHOD FOR MANUFACTURING COKE**
VERFAHREN ZUR SCHÄTZUNG DER KOKSSTÄRKE NACH REAKTION UND VERFAHREN ZUR HERSTELLUNG VON KOKS
PROCÉDÉ D'ESTIMATION DE LA RÉSISTANCE POST-RÉACTIONNELLE DU COKE ET PROCÉDÉ DE PRODUCTION DE COKE

(30) Priority: 02.11.2021 JP 2021179413
(43) Date of publication of application: 17.07.2024
(73) Proprietor: JFE Steel Corporation, Tokyo 100-0011 (JP)
(72) Inventor: TANDOKORO, Kohei, Tokyo 100-0011 (JP); ISHIDA, Tomoharu, Tokyo 100-0011 (JP); ARAKAWA, Sara, Tokyo 100-0011 (JP); YAMAMOTO, Tetsuya, Tokyo 100-0011 (JP)
(74) Representative: Hoffmann Eitle
(86) International application number: PCT/JP2022/034153
(87) International publication number: WO 2023/079837

(56) References cited:
- CN-A- 106 153 489
- JP-A- 2013 001 895
- JP-A- 2016 079 198
- JP-A- 2020 200 360
- US-A- 4 643 977

## Description

### Technical Field

The present invention relates to a method for rapidly estimating coke strength after reaction and a method for manufacturing coke utilizing such an estimation method.

### Background Art

When pig iron is manufactured by using a blast furnace, coke is an indispensable material as a reducing agent for iron ore. The primary reason for this is because coke, which is porous, has gas permeability such that a gas blown from the lower part of the blast furnace passes through the coke to the upper part of the blast furnace.

To ensure sufficient gas permeability, coke is required to have strength sufficient to suppress fracturing from occurring in the coke in a blast furnace. Usually, coke strength is often represented by a value such as rotating drum strength or the like which is obtained by performing a test in air at room temperature. However, since the interior of a blast furnace is in a high-temperature environment whose temperature is higher than 1000°C and which contains a gas such as carbon dioxide that is reactive with coke, the coke strength obtained on the basis of the results of the conventional test described above does not always correspond to the coke strength in the environment in a blast furnace.

As an index for accurately estimating the coke strength in a blast furnace, coke strength after CO₂ reaction (CSR) is proposed, and the blast furnace is operated on the basis of the CSR value. The analysis procedures of the CSR value are prescribed in ISO 18894 and ASTM D 5341. For example, in the case of the method prescribed by ASTM, after coke having a constant particle size has been held in a carbon dioxide atmosphere at a temperature of 1100°C for two hours, the coke is subjected to a rotating test utilizing an I-type drum tester, and the CSR value is defined as a lump residual ratio after a rotation number of 600. Usually, the lower limit of the CSR value is set, and daily operations are often performed while the CSR value is controlled not to be lower than the lower limit.

The most important issue regarding the CSR analysis described above is the time required. Although the prescribed reaction time between coke and CO₂ is two hours, since a large furnace is used, a long time is required for heating and cooling processes. Moreover, in the case where the time required for a series of operations such as sample preparation, evaluation based on rotating test after heating test, and the like is additive, the total time required may be almost one day. Even in the case where coke is subjected to the CSR analysis immediately after having been discharged from a coke oven, the coke is usually charged into a blast furnace before the analysis value is obtained. Therefore, since it is not possible to give an immediate response when an abnormal value is observed, there are potential risks of a delay in a corrective action and unstable blast furnace operation. Therefore, there is a demand for a method for rapidly evaluating the CSR value.

Regarding a method for rapidly evaluating the CSR value, various investigations have been conducted on the basis of methods for estimating the CSR value from the results of other analyses. In Patent Literature 1, by using Raman spectroscopy, an attempt is made to estimate the CSR value by utilizing the intensity ratio of a certain peak. Patent Literature 2 proposes a method for estimating the CSR value from the abundance ratios of inorganic constituents in coal.

In addition, investigations have also been conducted regarding a method for utilizing a coke reaction index (CRI), which has a high correlation with the CSR value. Patent Literature 3 discloses a method in which the CRI value of coke made from a coal blend is determined on the basis of the weighted average of the CRI values of relevant kinds of coke made from single coal brands and in which the CSR value of the coke made from the coal blend is estimated on the basis of the determined CRI value and drum strength of the coke made from the coal blend.

US 4 643 977 A discloses a process for assessing coke micro-reactivity.

However, to establish a method synchronized with the operation, a more rapid and simpler method for estimating the CSR value is required. In addition, particularly in the case of Patent Literature 2 and Patent Literature 3, since analysis values based on coal before being subjected to coking and reference samples prepared separately are used, there is also a problem in that it is difficult to take into consideration the effect of daily variance in practical operation of a coke oven.

### Citation List

### Patent Literature

PTL 1: Japanese Unexamined Patent Application Publication No. 2019-163986
PTL 2: Japanese Unexamined Patent Application Publication No. 2001-172643
PTL 3: Japanese Unexamined Patent Application Publication No. 2005-232350

### Summary of Invention

### Technical Problem

The present invention has been completed in view of the problem described above, and an object of the present invention is to provide a method for rapidly estimating coke strength after reaction.

### Solution to Problem

The present inventors diligently conducted investigations and, as a result, completed the present invention by focusing on the weight decrease rate of coke in the early stage of the reaction when the coke is analyzed by using a thermogravimetry (TG) method in a CO₂ atmosphere. The subject matter of the present invention is as follows.
[1] A method for estimating coke strength after reaction, the method including:
   a heating process of heating ground coke to a predetermined temperature range in an inert gas atmosphere and/or in a CO₂ atmosphere;
   a holding process of holding the heated coke in a CO₂ atmosphere at a holding temperature in the predetermined temperature range; and
   a process of determining a weight decrease rate of the coke after having been held at the holding temperature for a predetermined time by using a thermogravimetry method and of estimating coke strength after reaction from the weight decrease rate.
[2] The method for estimating coke strength after reaction according to item [1], in which, in the heating process, the coke is heated to a temperature range of 900°C or higher and 1150°C or lower at a heating rate of 20°C/min or higher and 150°C/min or lower.
[3] The method for estimating coke strength after reaction according to item [1] or [2], in which the predetermined time is 15 minutes or more and 25 minutes or less.
[4] A method for manufacturing coke, the method including estimating coke strength after reaction by using the method for estimating coke strength after reaction according to any one of items [1] to [3] and modifying conditions applied for manufacturing coke in accordance with the estimated coke strength after reaction.
[5] The method for manufacturing coke according to item [4], in which the modified conditions include coal blending ratios.

### Advantageous Effects of Invention

According to the present invention, it is possible to rapidly estimate coke strength after reaction (CSR) on the basis of the weight decrease rate of coke obtained by using a thermogravimetry (TG) method. Therefore, it is possible to expect to contribute to stabilizing blast furnace operation and a coal blending plan for manufacturing coke.

### Brief Description of Drawings

[Fig. 1] Fig. 1 is a diagram illustrating the part of the TG curve of common coke which is obtained when, in a CO₂ atmosphere, the coke is heated from room temperature at a heating rate of 40°C/min and held at a temperature of 1120°C for two hours.
[Fig. 2] Fig. 2 is a graph illustrating the relationship between the CSR value obtained by using a method in accordance with the ASTM standard and the weight decrease rate obtained by using the present invention. Description of Embodiments

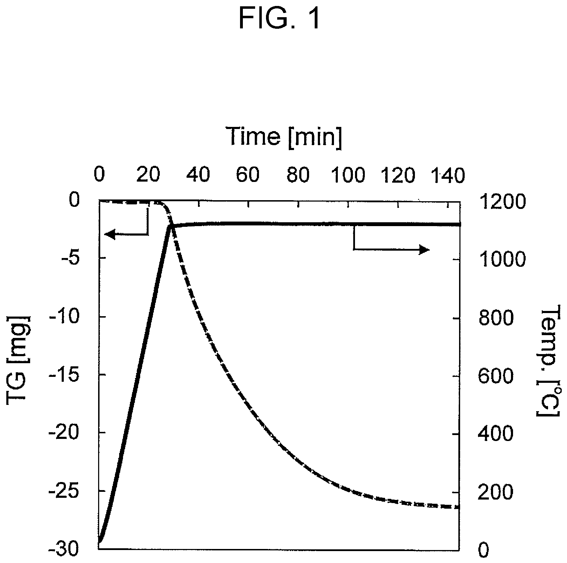

Fig. 1 is a diagram illustrating the part of the TG curve of common coke which is obtained when, in a CO₂ atmosphere, the coke is heated from room temperature at a heating rate of 40°C/min and held at a temperature of 1120°C for two hours. First, in the heating process of heating to a temperature of 1120°C, a minute weight change is observed. This is because of a weight increase due to inorganic materials contained in the coke in an amount of about 10 mass% absorbing CO₂ and a weight decrease due to the thermal decomposition of the coke.

In a range in which the holding temperature (1120°C in Fig. 1) is almost reached and holding is started, a sharp weight decrease starts. This is mainly because of a gasification reaction in which carbon reacts with CO₂ to form CO. That is, carbon which makes up the skeleton of coke is decomposed. Due to this, it is easy to assume that there is also a decrease in strength. Therefore, it is considered that such a range is a factor having an effect on the CSR value.

The present inventors analyzed such a TG curve and found that there is a correlation between a weight decrease rate in the early stage of the reaction and the CSR value. In particular, it was found that, by utilizing and analyzing a weight decrease rate in a time range from 15 minutes to 25 minutes from the start of holding at the holding temperature in the predetermined temperature range, in which the thermal decomposition of carbon is recognized on the TG curve, it is possible to use the analysis value as the index of the CSR value.

Hereafter, the specific embodiments of the present invention will be described. However, the present invention is not limited to the embodiments described below.

In the present invention, as an apparatus in which a coke sample is subjected to a thermal decomposition reaction test, a thermogravimetry (TG) apparatus is used. Since the TG apparatus is excellent in terms of atmosphere control and temperature responsivity, it is possible to rapidly measure a weight decrease rate due to the gasification reaction of coke in a CO₂ atmosphere.

A measurement atmosphere (of the holding process described below) is set to be a carbon dioxide (CO₂) atmosphere to simulate the interior of a blast furnace. This is the same environment as that used for the CSR analysis prescribed by ASTM and ISO. Here, in the CSR analysis, there may be a case where heating is performed in a nitrogen atmosphere. However, in the present invention, since it is confirmed that a difference in the atmosphere in which heating is performed in the CSR analysis causes only a small disturbance within an acceptable margin of error, the atmosphere in which heating is performed in the CSR analysis is set to be an inert gas atmosphere (of N₂, Ar, He, or the like) and/or a CO₂ atmosphere.

### (Coke)

In the present invention, ground coke is used. It is preferable that such coke be uniformly and finely powdered. In the case where the coke is not sufficiently ground, there is a risk that it is not possible to correctly measure the weight decrease rate due to variations in the distributions of the pores and inorganic materials in the coke. It is preferable that the particle size of the ground coke be 500 µm or less or more preferably 200 µm or less. The particle size of the ground coke may be adjusted by, for example, sieving the coke by using a sieve having a predetermined sieve opening.

The amount of coke used in the present invention may be adjusted in accordance with the capacity of the TG apparatus. To realize uniform heating, it is preferable that the above-mentioned amount of coke be 20 mg to 40 mg.

### (Heating process)

In the TG apparatus, the coke is heated to a predetermined temperature range in an inert gas atmosphere (of N₂, Ar, He, or the like) and/or a CO₂ atmosphere (heating process). That is, in the heating process, the coke is heated to a predetermined temperature range in any one of an inert gas atmosphere, a CO₂ (CO₂ gas) atmosphere, and an atmosphere of a mixture of an inert gas and CO₂. Here, in the heating process and the holding process described below, it is preferable that the atmosphere gas be fed into the TG apparatus at a flow rate of 200 mL/min or higher. In the case where the flow rate is lower than this, since the amount of gas fed is insufficient in relation to the amount of coke, there is a risk that it is not possible to correctly analyze the weight decrease rate due to a reaction.

### (Holding process)

Subsequently, holding is performed at a holding temperature (soaking temperature) in a predetermined temperature range (holding process). The holding process is performed in a CO₂ atmosphere. In the case where a gas which is different from CO₂ is used in the heating process described above, the atmosphere gas is switched to CO₂ gas at the same time as the transition to the holding process. It is preferable that the predetermined temperature range described above be 900°C or higher and 1150°C or lower. It is more preferable that the predetermined temperature range described above be 950°C or higher. It is more preferable that the predetermined temperature range described above be 1125°C or lower. In the case where the temperature range described above is lower than 900°C, since the weight decrease rate is low, it is difficult to recognize the difference between the samples. In addition, in the case where the temperature range described above is higher than 1150°C, since a decomposition reaction sharply progresses, there is a risk that an analysis error occurs. Although the heating rate to the temperature range described above (heating rate from room temperature to the temperature range described above) may be appropriately adjusted in accordance with the capacity of the TG apparatus, it is preferable that the heating rate be 20°C/min or higher and 150°C/min or lower. In the case where the heating rate is lower than 20°C/min, since a long time is required for the temperature range described above to be reached, there is a decrease in the contribution to a method for rapid analysis. On the other hand, in the case where the heating rate is higher than 150°C/min, since the portion of the TG curve corresponding to the decomposition reaction of inorganic materials, which occurs in a lower temperature range, and the portion of the TG curve corresponding to the decomposition reaction of the coke overlap each other due to the sharp temperature change, there is a risk that it is difficult to correctly determine the weight decrease rate. Here, room temperature is, for example, 25°C.

### (Process of estimating coke strength after reaction)

Subsequently, the weight decrease rate of the coke after having been held at the holding temperature (soaking temperature) in a predetermined temperature range for a predetermined time is determined by using a thermogravimetry method. In addition, coke strength after reaction is estimated from the determined weight decrease rate.

It is preferable that the predetermined time described above be 15 minutes or more and 25 minutes or less, which is sufficient for a sample (coke) charged into a TG apparatus to react. More in detail, it is preferable that the predetermined time be 15 minutes or more and 25 minutes or less after holding at the holding temperature in a predetermined temperature range has been started in the holding process. In the case where the predetermined time is less than 15 minutes, since the thermal decomposition of inorganic materials and the like are included in the decomposition reaction, there is a risk that an analysis error occurs. In addition, in the case where the predetermined time is more than 25 minutes, since the decomposition reaction of coke progresses sufficiently, the weight decrease rate is low. Therefore, since it is difficult to recognize the difference in the weight decrease rate, there is a risk that an analysis error occurs.

In addition, the weight decrease rate is defined as the amount of weight decrease (the amount of decrease in weight) per unit time of the sample (coke) when the sample is held at the holding temperature in the predetermined temperature range for the predetermined time. It is preferable that the weight decrease rate be defined as the amount of weight (mg) decrease per unit time (min) of the sample (coke) when the sample is held at the holding temperature in the predetermined temperature range for 15 minutes or more and 25 minutes or less. It is more preferable that the weight decrease rate be defined as the amount of weight (mg) decrease per unit time (min) of the sample (coke) when the sample is held at the holding temperature in the predetermined temperature range in a period from when 15 minutes has elapsed until 25 minutes has elapsed after holding at the holding temperature in the predetermined temperature range has been started in the holding process [(amount of change (decrease) in weight (mg))/(10 (min)) of the coke when the coke is held at the holding temperature in the predetermined temperature range in a period from when 15 minutes has elapsed until 25 minutes has elapsed after holding at the holding temperature in the predetermined temperature range has been started]. The weight decrease rate of the coke defined as described above has a high correlation with the CSR value. That is, in the case where the weight decrease rate is low, since the decomposition reaction of coke does not occur, the CSR value is high because the coke strength is maintained. In addition, by utilizing such a correlation, it is possible to estimate the CSR value of the coke from the weight decrease rate obtained by using a thermogravimetry method. Specifically, for example, by determining in advance the correlation between the weight decrease rate obtained by using a thermogravimetry method and the CSR value obtained by using a method in accordance with the prescription by ASTM regarding plural kinds of coke, and by determining, by using a thermogravimetry method, the weight decrease rate of target coke, whose CSR value is to be estimated, it is possible to estimate the CSR value of the target coke from the determined weight decrease rate and the correlation determined in advance.

In the method for estimating coke strength after reaction according to the present invention, by utilizing the weight decrease rate of coke when the coke is held at the holding temperature in the predetermined temperature range for the predetermined time, it is possible to estimate the CSR value within about two to three hours. Therefore, it is also possible to assess the CSR value in real time during coke oven operation.

In a practical application, in the case where the estimated coke strength after reaction is out of the operationally acceptable range of coke strength after reaction, it is possible to stop feeding the coke into a blast furnace without delay, which results in stable blast furnace operation. In addition, since it is also possible to efficiently control the coal blending ratios of coke by, for example, modifying coal blending ratios in accordance with the estimated coke strength after reaction, it is also possible to optimize the coal blending ratios. In addition, since it is also possible to modify conditions applied for manufacturing coke without delay in accordance with the modified coal blending ratios, it is possible to improve the quality of coke and to stabilize blast furnace operation. Here, the modification of the conditions applied for manufacturing coke in accordance with the coke strength after reaction corresponds to the "method for manufacturing coke" according to the present invention. In addition, the modification of conditions applied for manufacturing coke includes the modification of the coal blending ratios in accordance with the coke strength after reaction.

### EXAMPLE 1

Hereafter, the present invention will be described in accordance with examples. However, the present invention is not limited to the examples below.

13 kinds of coke (Nos. 1 to 13) given in Table 1 were prepared. The CSR value of each kind of coke was calculated by using the method prescribed by ASTM. The results are given in Table 1. Here, the drum strength index DI of each kind of coke is also given in Table 1 for reference.

Separately, a sample was prepared by weighing out 20 g of each kind of coke, and the whole volume of the sample was ground and sieved so as to have a particle size of 150 µm or less. After the sieved sample had been subjected to air drying, a specimen was prepared by weighing out 30 mg of the dried sample. As a TG apparatus, Thermo Plus 2 TG8120 produced by RIGAKU Corporation was used. The prepared specimen was packed in a container made of platinum, which was attached to the apparatus, and charged into the apparatus. The atmosphere gas was CO₂, and the gas flow rate was 300 mL/min. After the apparatus had been stabilized, the specimen was heated from room temperature to a temperature of 1100°C at a heating rate of 40°C/min, held at a temperature of 1100°C for 30 minutes, and then naturally cooled to room temperature. With reference to the TG curve obtained by the TG measurement, a weight decrease rate in periods of 5 minutes (10 minutes in total) before and after the time when 20 minutes had elapsed after heating to a temperature of 1100°C had been completed and holding at a temperature of 1100°C had been started was determined. That is, the amount of coke weight decrease per unit time in a period from when 15 minutes had elapsed until 25 minutes had elapsed after holding at a temperature of 1100°C had been started [(difference (mg) between the amount of weight decrease after 15 minutes had elapsed and the amount of weight decrease after 25 minutes had elapsed)/10 (min)] was determined.

**[Table 1]**

| No. | DI | CSR |
|---|---|---|
| 1 | 85.4 | 60.1 |
| 2 | 85.7 | 60.9 |
| 3 | 86.1 | 58.8 |
| 4 | 86 | 56.4 |
| 5 | 85.4 | 62.1 |
| 6 | 85.8 | 65.9 |
| 7 | 85.8 | 63.7 |
| 8 | 85.9 | 64.3 |
| 9 | 86 | 67.7 |
| 10 | 85.4 | 68.9 |
| 11 | 85.5 | 66.2 |
| 12 | 86.4 | 66.2 |
| 13 | 85.7 | 63.6 |

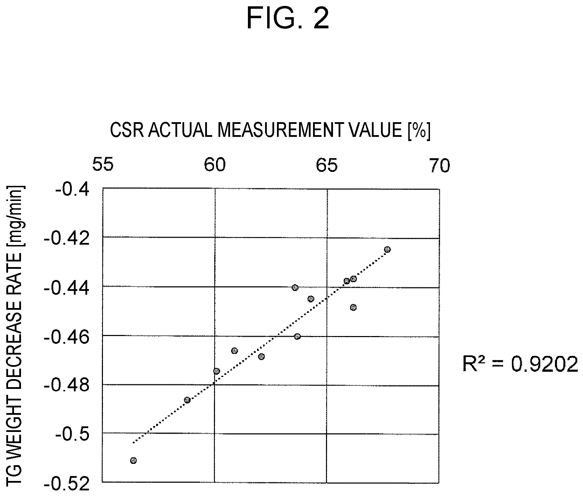

The relationship between the weight decrease rate obtained as described above and the CSR value obtained by using a method in accordance with the ASTM standard is illustrated in Fig. 2. It is clarified that there is a high correlation between the two. That is, it is possible to estimate the CSR value from the weight decrease rate determined from the TG as described above. While it may take half a day or more or about 24 hours in some cases in total in the case of the method prescribed by ASTM, that is the conventional method, it is possible to estimate the CSR value in about 3 hours in the case of the method for estimating coke strength after reaction according to the present invention.

## Claims

1. A method for estimating coke strength after reaction, the method comprising:
a heating process of heating ground coke to a predetermined temperature range in an inert gas atmosphere and/or in a CO₂ atmosphere;
a holding process of holding the heated coke in a CO₂ atmosphere at a holding temperature in the predetermined temperature range; and
a process of determining a weight decrease rate of the coke after having been held at the holding temperature for a predetermined time by using a thermogravimetry method and of estimating coke strength after reaction from the weight decrease rate.

2. The method for estimating coke strength after reaction according to Claim 1, wherein, in the heating process, the coke is heated to a temperature range of 900°C or higher and 1150°C or lower at a heating rate of 20°C/min or higher and 150°C/min or lower.

3. The method for estimating coke strength after reaction according to Claim 1 or 2, wherein the predetermined time is 15 minutes or more and 25 minutes or less.

4. A method for manufacturing coke, the method comprising estimating coke strength after reaction by using the method for estimating coke strength after reaction according to any one of Claims 1 to 3 and modifying conditions applied for manufacturing coke in accordance with the estimated coke strength after reaction.

5. The method for manufacturing coke according to Claim 4, wherein the modified conditions include coal blending ratios.

## Patentansprüche

1. Verfahren zur Schätzung der Koksstärke nach Reaktion, wobei das Verfahren umfasst:
einen Heizprozess zum Erhitzen von gemahlenem Koks in einer Inertgasatmosphäre und/oder in einer CO₂-Atmosphäre auf einen vorbestimmten Temperaturbereich;
einen Halteprozess zum Halten des erhitzten Koks in einer CO₂-Atmosphäre bei einer Haltetemperatur im vorbestimmten Temperaturbereich; und
einen Prozess zum Bestimmen einer Gewichtsabnahmerate des Kokses, nachdem dieser für eine vorbestimmte Zeit bei der Haltetemperatur gehalten wurde, unter Verwendung eines Thermogravimetrieverfahrens, und zur Schätzung der Koksstärke nach Reaktion aus der Gewichtsabnahmerate.

2. Verfahren zur Schätzung der Koksstärke nach Reaktion nach Anspruch 1, wobei der Koks im Heizprozess mit einer Heizrate von 20 °C/min oder mehr und 150 °C/min oder weniger auf einen Temperaturbereich von 900 °C oder mehr und 1150 °C oder weniger erhitzt wird.

3. Verfahren zur Schätzung der Koksstärke nach Reaktion nach Anspruch 1 oder 2, wobei die vorbestimmte Zeit 15 Minuten oder mehr und 25 Minuten oder weniger beträgt.

4. Verfahren zur Herstellung von Koks, wobei das Verfahren eine Schätzung der Koksstärke nach einer Reaktion unter Verwendung des Verfahrens zur Schätzung der Koksstärke nach Reaktion nach einem der Ansprüche 1 bis 3, und Modifizieren von zur Herstellung von Koks angewandten Bedingungen entsprechend der abgeschätzten Koksstärke nach einer Reaktion umfasst.

5. Verfahren zur Herstellung von Koks nach Anspruch 4, wobei die modifizierten Bedingungen Kohlemischungsverhältnisse einschließen.

## Revendications

1. Procédé d'estimation de la résistance post-réactionnelle du coke, le procédé comprenant :
un processus de chauffage consistant à chauffer le coke broyé jusqu'à une plage de température prédéterminée sous atmosphère de gaz inerte et/ou atmosphère de CO₂ ;
un processus de maintien du coke chauffé sous atmosphère de CO₂ à une température de maintien comprise dans la plage de températures prédéterminée ; et
un processus de détermination d'un taux de diminution de poids du coke après avoir été maintenu à la température de maintien pendant un temps prédéterminé en utilisant une méthode de thermogravimétrie et d'estimation de la résistance post-réactionnelle du coke après réaction à partir du taux de diminution de poids.

2. Procédé d'estimation de la résistance post-réactionnelle du coke selon la revendication 1, dans lequel, dans le processus de chauffage, le coke est chauffé jusqu'à une plage de températures comprise entre 900 °C et 1.150 °C à une vitesse de chauffage comprise entre 20 °C/min et 150 °C/min.

3. Procédé d'estimation de la résistance post-réactionnelle du coke selon la revendication 1 ou 2, dans lequel le temps prédéterminé est de 15 minutes ou plus et de 25 minutes ou moins.

4. Procédé de production de coke, le procédé comprenant l'estimation de la résistance post-réactionnelle du coke en utilisant le procédé d'estimation de la résistance post-réactionnelle du coke selon l'une quelconque des revendications 1 à 3 et la modification des conditions appliquées à la fabrication du coke en fonction de la résistance post-réactionnelle estimée du coke.

5. Procédé de production de coke selon la revendication 4, dans lequel les conditions modifiées incluant les proportions de mélange de charbon.
